# EUROPEAN PATENT APPLICATION

(11) **EP 1 982 699 A1**
(43) Date of publication of application: **22.10.2008**
(21) Application number: 07712570.6
(22) Date of filing: 19.01.2007
(51) Int. Cl.: A61K 9/19, A61K 31/337

(54) **A SOLID LYOPHILIZED TAXANE COMPOSITION, A METHOD FOR PREPARING SAID SOLID COMPOSITION, A PHARMACEUTICAL FORMULATION AND A KIT FOR SAID FORMULATION**

(30) Priority: 20.01.2006 AR P060100208
(71) Applicant: Eriochem, S.A., Parana, Entre Rios (AR)
(72) Inventor: BOUZADA, Antonio Osvaldo, Paraná, E3102FLD (AR); NUÑEZ, José Lucio, Paraná - Entre Rios, E3100FGD (AR); ITURRASPE, José Bernardo, Santa Fe - Santa Fe, S3002FOE (AR); MOYANO DE ITURRASPE, Nora Adriana, Santa Fe - Santa Fe, S3002FOE (AR)
(74) Representative: Gislon, Gabriele
(86) International application number: PCT/ES2007/070013
(87) International publication number: WO 2007/082979

(57) **Abstract**

A lyophilised solid composition of taxane (preferably docetaxel and paclitaxel), suitable to prepare pharmaceutical formulation to be administered to mammals, particularly humans, comprising said taxane, a tensoactive, lyophilizing excipient and acid; also essentially free from organic solvents. Said solid composition is free from polysorbate 80 and polyoxyethylated castor oil; it is sterile; it is soluble in aqueous solutions in the absence of organic solvent and it has an apparent density from 0.05g/ml and 0.45g/ml. A procedure of double lyophilisation to obtain said solid composition of taxane. A pharmaceutical formulation of a taxane which comprises said solid composition of lyophilized taxane and a solubilizing composition. A kit comprising said compositions, and a syringe.

## Description

### Field of the invention

This invention belongs to the field of the formulations of pharmaceutical drugs which are poorly soluble in water. Particularly, it refers to oncological drug formulations, in which said drugs belong to the taxane group, and procedures to obtain said drugs. More specifically, the invention is directed to formulations intended for parenteral infusion processes typical of oncological chemotherapy with docetaxel and paclitaxel.

### State of the art

The pharmaceutical formulations of drugs which are poorly soluble in aqueous media have been extensively studied over the last decades. Countless strategies have been developed in order to inject these drugs into mammals with the aim of improving their pharmacotechnical properties and ameliorate their side effects.

Concerning formulations which are suitable for the preparation of solutions for parenteral infusion over a long period, and especially for oncological chemotherapy treatments, technical problems arise, namely as how to maintain these drugs in the aqueous solution of the parenteral infusion for periods of at least 4 hours for conventional infusion protocols and at least for 72 hours for administration by means of a continuous infusion pump.

Of a particular interest for this invention are the compounds of the taxane family, products extracted from the leaves and bark of a tree commonly known as European yew (*Taxus Baccata* and other species of the Taxus family) as paclitaxel, as well as semi-synthetic products obtained from baccatin III or from 10-deacetylbaccatin III which are also extracted from yew, like docetaxel. Those taxanes obtained from biotechnological processes are also of interest for the present invention. The medical uses of taxanes are varied; their anti-tumoral effects can be mentioned among others. Some examples of cancer which can be treated with docetaxel are: locally advanced or metastatic breast cancer, non-small cell lung cancer, hormone-refractory prostate cancer and ovarian cancer, and gastric cancer.

Patent US4814470 to Colin, Michel et al., from the company Rhône-Poulenc Santé (Courbevoie, FR), refers to a taxane pharmaceutical composition from which 10-deacetylbaccatin III derivatives are obtained, especially docetaxel. This document describes a synthesis that ends with a crystallisation and a formulation comprising the dissolution of the crystals obtained in a mixture of equal parts of non-ionic tensoactives and alcohol. This formulation gellifies when injected into the parenteral infusion bag.

A series of patents from the company Rhône-Poulenc Santé (Courbevoie, FR) protects the current formulation of docetaxel (US5438072, US5698582, US5714512 and US5750561). This technology solves the *in situ* precipitation and gellification problems by a formulation having two solutions: one of taxane in polysorbate 80 and alcohol (which can be present in very low concentrations) and the other of water and ethanol. These documents describe a preparation procedure which consists of mixing both solutions without intense agitation, to generate a solution of Taxotere^{™} 10mg/ml (stable between 2 and 25°C for 4 hours), which solves the problem of gellification *in situ* by diluting such solution in an aqueous 5% dextrose solution or normal saline solution of sodium chloride at 0.9% to obtain the infusion solution at a concentration of 0.3 to 0.74mg/ml (stable for 8 hours, between 2 and 25°C).

This liquid formulation of docetaxel in polysorbate 80 (in the presence or absence of alcohol) poses the problem of stability in time; being a room temperature between 2 and 25°C recommended for its conservation. Besides, the presence of polysorbate 80 causes known adverse side effects due to the incorporation of said tensoactive in high concentrations -which are necessary to maintain the drug in solution- into the blood stream. The same happens with the formulation of paclitaxel available on the market, which requires high concentrations of Cremophor^{™}. The adverse side effects caused by these tensoactives present in taxane formulations currently available, particularly docetaxel and paclitaxel, require a pre-treatment with steroids or antihistamines before oncological chemotherapy as described in the literature, as follows: 'ten Tije AJ, Verweij J, Loos WJ, Sparreboom A. Pharmacological Effects of Formulation Vehicles: Implications for Cancer Chemotherapy. Clin Pharmacokinet 2003; 42: 665-685'; 'Rowinsky EK, Eisenhauer EA, Chaudhry V et al. Clinical toxicities encountered with paclitaxel (Taxol). Semin Oncol 1993, 20: 1-15'; 'Bernstein B. Docetaxel as an Alternative to Paclitaxel after Acute Hypersensitivity Reactions. Ann Pharmacother 2000; 34:1332-1335'; 'Novel Formulations of Taxanes: A review. Old Wine in a New Bottle? K. L. Hennenfentl & R. Govindan. Annals of Oncology 17: 735-749, 2006 doi: 10.1093/annonc/mjd 100 Published on line 19 December, 2005'.

Likewise, the formulation of docetaxel currently available on the market requires for its use a procedure which involves several steps and a certain risk for the doctors and nurses involved in its administration. Said steps, aimed at ensuring the proper administration of this drug involve: extracting the solvent from an ampoule and introducing it into a vial containing docetaxel solution, gently agitating to homogenize the solution, allowing a rest time for foam to disappear, extracting the mix and injecting it into the perfusion bag or flask filled with saline solution or dextrose, homogenizing and finally inspecting it for possible precipitate formation before administering it to the patient (should precipitation appear, the solution must be disposed of with the resulting economical negative impact).

The whole procedure must be carried out aseptically. The risks of contamination are high and the operation requires trained personnel and a considerable time. There is also a risk of contamination for health care providers in contact with the cytotoxic solution handled, due to the aerosolisation of the drug.

Currently, there is a need to simplify said process of administration, to shorten the time for the preparation of the perfusion formulation and to decrease the risks implied.

The toxic effects of the tensoactives used in the formulation of docetaxel and paclitaxel, such as polyoxyethylated castor oil (Emulphor^{™} or Cremophor^{™}) or polysorbate 80 (Tween 80™ ) are known. The pharmacological and biological effects caused by these tensoactives, such as acute hypersensitivity reactions, peripheral neuropathy, cumulative fluid retention syndrome, etc. have been described. A great number of patients cannot be treated due to the side effects of such tensoactives, eg. patients presenting hypersensitivity, patients with impaired renal function, elderly patients, patients suffering from a cardiopathy, etc. These tensoactives also affect the availability of the drugs which are solubilised and administered intravenously.

That is the reason why great efforts have been made in the scientific field in order to find formulations which prevent or decrease the use of said tensoactives. Among the strategies described in the state of the art, we can mention the development of albumin nanoparticles, polyglutamates, taxane analogs, prodrugs, emulsions, liposomes, etc.

For the purpose of reference, literature is provided to offer a detailed description of the latest development and clinical tests: 'Novel formulations of taxanes: A Review. Old Wine in a New Bottle?' K. L. Hennenfentl & R. Govidan 21st Louis college Pharmacy. Ortho Biotech Clinical Affairs, LLC, St Louis MO; 2 Alvin J Siteman Cancer Center, Washington University School of Medicine, St. Louis, MO, USA accepted 7 November, 2005. Other works contributing solutions to these problems are: 'Castro CA et al. Pharmacol Biochem Behav, 1995 Apr; 50(4):521-6; Sanzgiri UY et al., Fundam Appl Toxicol, 1997 Mar; 36(1):54-61; ten Tije AJ et al., Clin Pharmacokinet. 2003; 42(7):65-85':' Constantine JW et al., Experientia. 1979 Mar 15;35(3):338-9':' Van Zuylen L et al., Invest New Drugs 2001 may;19(2):125-41':' Bergh M et al., Contact Dermatitis. 1997 Jul; 37(1):9-18)'.

The World Health Organization has estimated that the maximum daily dose of polysorbate 80 is 25mg/kg of body weight (FAO WHO. Tech. Rep. Ser. Wld. Hlth. Org. 1974, N° 539). Therefore, a man weighing 75 kg may be administered 1.875 g of polysorbate 80 per day; i.e., for a dosage form of 75 mg of Taxotere^{™} per m² commonly used for lung or prostate cancer, a man who is 1.75m tall and weighs 75 kg, has a body surface of 1.90 m² and is to be administered 143 mg of docetaxel along with approximately 3.6 g of polysorbate 80 (40 mg of docetaxel per ml of Tween 80™ i.e. almost 2-fold the maximum daily dosage form of polysorbate 80 recommended by the WHO.

On the other hand, lyophilised formulations of certain injectable drugs tend to be more advantageous than injectable liquid formulations, particularly in those cases in which the lyophilised solution is of a higher chemical and physical stability, i.e, it has a longer shelf-life and is more resistant to higher temperatures, as those in the warm climates of the regions belonging to Zone 4, according to the International Committee for Harmonization (ICH).

Particularly, the process of lyophilisation of drugs which are poorly soluble in water, mainly taxanes, presents great difficulties because the standard techniques of lyophilisation consist of freezing aqueous solutions and subjecting them to vacuum to achieve sublimation. Apart from water there are not many solvents which allow this procedure within acceptable pharmacotechnical conditions. In order to be lyophilised, a good formulation should not pose "puffing" problems, i.e, when frozen it should not generate a plastic solid which bubbles on sublimation. Furthermore, it should be a good heat conductor and the solvent should generate a lyophilised cake of suitable pharmacotechnical characteristics. Moreover, the material structure should meet the condition of having spaces to allow the diffusion of the gas produced by the sublimation of the solvent through the cake. Besides, said cake should be rigid enough to support its own structure, aided by an excipient if needed.

There have been many attempts in the state of the art in order to solve the problem of toxicity of non-ionic tensoactives, proposing taxane lyophilised formulations. For example, patent WO 99/24073 to Géczy, J (Thissen Laboratories S.A.) proposes to lyophilise docetaxel and paclitaxel starting from a hydroalcoholic solution of these drugs and cyclodextrins. This solution allows obtaining a lyophilisate containing taxane complexed to cyclodextrin (it uses 2hydroxypropyl β-cyclodextrin for docetaxel in a mass ratio of approximately 1:100 of active principle: cyclodextrin). This lyophilisate can be dissolved in an aqueous solution of up to 1mg/ml, ready for perfusion. Although this eliminates the use of non-ionic tensoactives, this formulation incorporates into the blood stream a complex of taxane and cyclodextrin which can modify the significant pharmacodynamic properties of taxane. Therefore, a toxicological and clinical study is required to endorse the use of this new complex between taxane and cyclodextrin. Besides, it involves complex elaboration processes.

Other attempts to obtain taxane lyophilisates are shown in the state of the art, such as the application for patent US2003/0099674 by Chen, Andrew in which obtaining a lyophilised taxane from an oil/water emulsion using lecithin as a tensoactive and sucrose as an anti-adhesion agent is proposed. Bile salts are among the surfactants mentioned in the description. Taxane is dissolved in ethanol and water, and the solvents are then eliminated by lyophilisation, generating a taxane liposome when it is reconstituted with water.

Furthermore, the application of patent US2003/0099675 by Jeong, Seo Young, proposes a liquid formulation having an organic solvent, an emulsifier and a monoglyceride. It also proposes forming an emulsion in water from a liquid formulation and lyophilising it. The use of active drugs such as paclitaxel is mentioned in this document.

In these last two applications a taxane lyophilisate with a good solubility in water is obtained. However, said taxane lyophilisate poses similar problems to those mentioned before as regards the use of taxane emulsions which can modify their pharmacodynamics. Naturally, emulsions and microemulsions as liposomes generate autoimmune responses when administered endovenously and are attacked by macrophages, which causes an important part of the dosage to be unavailable for the desired action, apart from generally requiring a pretreatment with steroids or antihistamines.

Other technological development as the one described in the application US2003/0187062 by Zenoni Mauricio, et al., from ACS DOBFAR S.A., proposes obtaining micro or nanoparticles by lyophilising paclitaxel and albumin.

There is a vast literature describing taxane liposomes as in patent EP1332755, in which a lyophilisate of paclitaxel is obtained using a compound like lecithin or cholesterol in a solution of isopropanol or ethanol in order to subsequently obtain liposomes which are always lyophilised from aqueous solutions. One of the drawbacks of these developments is that they modify the pharmacodynamics of taxane, as they have a short useful life and require a cold chain for their preservation, apart from generating an immune response and causing the attack of macrophages which decreases the effect of the drug considerably.

There are many patents claiming polymer micellae in the literature, namely patents US5543158 and US6322805. In particular, patent US6322805 refers to obtaining biodegradable polymeric micellae capable of solubilising hydrophobic drugs comprising amphiphillic block copolymers, which have a hydrophilic polyalkyl oxide and a biodegradable hydrophobic polymer selected from the group consisting of polylactic acid, polyglycolic, polylactic-co-glycolic acid, poly(epsilon-caprolactone) derivatives and the mixtures thereof. It describes how the hydrophobic drug is trapped in the micelle in the absence of a covalent bond. These micellae form a solution in water acting as solubilising agent. This solution can be lyophilised, preserved and reconstituted with water or isotonic solution. This patent does not solve the problem of increasing the solubility of taxane as such, but it presents a complex process of synthesis of a specific copolymer to generate taxane micellae. In spite of having done promising tests regarding the characteristics of stability, components are added to the drug thus radically changing not only the bioavailability but also the kinetics of the original taxane. This patent does not describe the method to obtain a sterile solution by means of reconstitution.

US Patent N° 6780324 describes a process in which a solution of a biologically active hydrophobic agent is formed in combination with a dispersing agent and an organic solvent or even a mixture in which water can be included. This mixture can be lyophilised and rehydrated to form a nanodispersion or a micelle solution. Drug is not lost during this process; it can be sterilised by filtration. A transparent solution is obtained by reconstitution but the lyophilisate does not have an agent to ensure taxane stability as an acid. Besides the proposed lyophilisation does not ensure that the final solid formulation is essentially free from proposed organic solvents such as t-buthanol, n-buthanol, dioxane, pyridine, pirimidine and piperidine, which could cause adverse side effects if administered to humans. On the other hand, the procedure proposed requires operations such as sonication, intense stirring or heating to obtain the solution to be lyophilised which can either destroy micellae or cause taxane degradation.

US Patent N° 6610317 B2 to Julie Straub et al. describes a porous matrix of paclitaxel produced by mixing taxane dissolved in organic solvent, specifically ethanol, with polysorbate 80, other tensoactives and excipients like mannitol to further evaporate the solvent by spray drying. This formulation proposes a soluble solid having components in its formulation such as polysorbate 80 which can cause undesirable side effects. Besides, it proposes heating the drug for its drying, a step which originates degradation products known by the art. On the other hand, the paclitaxel solution that can be produced following the teachings of said patent contains 80% ethanol, which would make the process of lyophilisation not possible to apply in order to generate the solid formulation proposed.

The application of patent US2004/0247624 A1 by Unger, Evan Charles, et al. describes another method to formulate drugs which are poorly soluble in water. It proposes to elaborate a solid composition lyophilising a filtered solution of an organic solvent such as terbutanol, ciclohexane, dimethyl carbonate, dimethyl sulphoxide and acetic acid, the poorly soluble drug and at least a stabilising agent which does not have a covalent bond with the drug. The only example with paclitaxel (example #3) describes a complex process of sonication and heating of a mixture of two polymers in high concentration, t-buthanol and the drug until solubilisation is reached. Subsequently lyophilisation is proposed to obtain a powder. Using the solvents proposed in this application as lyophilisation solvents, there always remain significant concentrations of said solvents, incorporated to the lyophilised solids, even when heated for long periods. Finally, it describes redissolution of said powder with another tensoactive solution which finally cannot dissolve the whole solid as it states that there are visible particles. Moreover, heating the drug in the solution at 60°C causes the degradation of paclitaxel. On the other hand, it does not contain any taxane stabiliser, therefore said formulation has the risk of degrading.

Application US2005/0152979 by Besman, Marc et al. claims a lyophilised composition of a drug poorly soluble in water which contains said drug, a polymer and an agent to improve reconstitution. In spite of claiming paclitaxel and docetaxel as drugs suitable to be used by the invention, the assays it states are performed with a paclitaxel conjugate named CT-2103 which is an ester of paclitaxel and alpha-poly-(L)glutamic acid, of a much higher solubility in aqueous solutions than paclitaxel itself. Neither paclitaxel nor docetaxel dissolves in sodium phosphate aqueous solutions with the excipients and tensoactives described in this document.

Other technologies like those used in taxane conjugates are described in documents such as the application of patent US2003/0147807 by Chun Li et al. which describes a taxane composition soluble in water. However, it refers to conjugates of paclitaxel and docetaxel bound to soluble polymers such as polyglutaminic acid, polyaspartic acid, etc.

Other patents evaluated, which are not relevant to the object of the present invention but which are nevertheless part of the state of the art in this technological field are the following: US2005/0191323, WO9814174, US6630121, US6607784, WO2005/044225, US2006/0127420, US2006/0078619, US2004/0091541, US2003/0215496, US2001/0018072, US5922754 and WO2005/025499.

The current state of the art offers a great number of solutions to obtain formulations of drugs of a low solubility in water. However, it was not possible to obtain in said solutions a lyophilised sterile solid composition of taxane, in particular docetaxel, and free from other residual organic solvents which also has a certain concentration of acid ensuring chemical stability. It has neither been found, in the state of the art, a procedure of double lyophilisation allowing to take advantage of the solubilising capacity of the lyophilising organic solvents to eventually obtain a solid composition of lyophilised taxane free from said solvents, and soluble in water and aqueous solutions in the absence of organic solvents.

To achieve the solubilisation of these drugs of a poor solubility in aqueous media, a great number of methods have been proposed in the state of the prior art, such as intense agitation, heating, sonication, solvent evaporation, dialysis, spray-drying, emulsification/evaporation, micronisation, etc. The proposals from the prior state of the art, which involved lyophilisation as part of the procedure, generally start from a mixture containing organic solvents, water, polymers, excipients, tensoactives, lipids, and lipoproteins among other components. These mixtures usually involve complex emulsification or dilution processes that often require heating, sonication, intense agitation and use of polymers specially designed, among other procedures.

The prior art describes, in general, the dissolution of taxanes in an organic solvent, generally ethanol and subsequently other components are added namely tensoactives, water, excipients, etc. No literature has been found so as to infer that paclitaxel or docetaxel as such are solubilised in an aqueous solution in the absence of an organic solvent added to it.

Furthermore, it has been proved that the presence of organic solvents such as ethanol enhances the solubility of taxanes but affects their stability favouring anticipated precipitation of the drug when formulated in an aqueous solution of perfusion. Therefore, it would be convenient to develop a formulation of taxanes free from organic solvents.

The present invention proposes a pharmaceutical composition free from organic solvents, polysorbate 80 and polyoxyethylated castor oil, which makes it unnecessary the usual pre-treatment with antihistamines or steroids in oncological therapy with taxanes, and also reduces the administration time per perfusion.

A great number of adverse effects are attributed to polysorbate 80; therefore this new solid composition of lyophilised taxane allows for the preparation of a formulation of perfusion which can be administered to patients who cannot receive it nowadays, namely patients with renal illnesses, cardiopathies, those with polysorbate 80 hypersensitivity, etc. It can also be further administered to patients suffering from kidney cancer as it does not contain polysorbate 80 which causes fluid retention syndrome.

The technologies using organic solvents such as terbutanol, ciclohexane, dimethyl carbonate, dimethyl sulphoxide, dioxane or acetic acid to manufacture lyophilised taxanes does not solve the problem of the residual presence of said solvents in the lyophilized powder, which are not eliminated even by heating for long periods. It is known that some of said organic solvents might pose toxicity problems, and could be the cause of adverse reactions if said lyophilised compositions were administered to humans. Said lyophilized taxanes do not conform to norms such as ICH Q3C which allows residual solvents in pharmaceutical solid products of up to 0.5% of acetic acid and up to 0.3%of dioxane, for example.

Besides, lyophilisers used at pharmaceutical plants are prepared to lyophilise aqueous solutions and therefore they are in general made of stainless steel, which allows sterilisation with vapour as required by current norms. Said lyophilisers are not prepared to work with organic solvents as those mentioned in the paragraph above. Said organic solvents are toxic, corrosive, flammable or explosive. For example, acetic acid is a corrosive and solvent agent as dioxane, terbutanol and dimetylsufoxide. They require additional infrastructure for gas treatment due to their toxicity, in order to avoid accidents and the contamination of personnel during production. Said infrastructure is very expensive and of a complex implementation in pharmaceutical lyophilisers of final products. On the contrary lyophilisers of production of active ingredients of bulk pharmaceutical products require less demanding sanitizations than vapour sterilisation. Also, bulk lyophilisation requires much smaller lyophilisers than the equipment used for the lyophilisation of final pharmaceutical products in vials. As a consequence, the lyophilisation of pharmaceutical products, lyophilised from solutions containing said organic solvents poses an operational problem of complex and costly solution. The present invention solves such problem: it proposes bulk lyophilisation in a lyophiliser of production of pharmaceutical active ingredients, of a taxane solution with an organic lyophilisation solvent, resulting in a pharmaceutical active of an improved solubility, which is lyophilised in aqueous solutions free from organic solvents and is lyophilised in vials, in a pharmaceutical lyophiliser for final products. Thus, the benefits of said organic solvents of lyophilisation are taken advantage of and at the same time a pharmaceutical final product free from said solvents is obtained, using a standard pharmaceutical lyophiliser without modifications, for aqueous solutions are processed.

### Summary of the invention

The first object of the present invention is to provide a lyophilised solid composition of taxane, suitable for the preparation of pharmaceutical formulations for mammals, in particular humans, which comprises said taxane, a tensoactive, lyophilisation excipients and acid, and which is also essentially free from organic solvent, wherein said solid composition is free from polysorbate 80 and polyoxyethylated castor oil; it is sterile; it is soluble in aqueous solutions in the absence of an organic solvent; it has an apparent density from 0.05 g/ml to 0.45 g/ml; and preferably an apparent density from 0.10 g/ml to 0.35g/ml.

A second object of the present invention is to provide a procedure to prepare said lyophilised taxane solid composition comprising the following steps:
a) dissolving said taxane in lyophilisation organic solvent
b) lyophilising
c) dissolving the lyophilised taxane from the previous step in an aqueous solution
d) lyophilising the aqueous solution of taxane obtained in step c)
wherein said organic lyophilising solvent is selected from the group comprising dioxane, acetic acid, dymetylsuphoxide and the mixtures thereof; preferably dioxane or acetic acid. Moreover, said taxane from step a) dissolves only in said organic lyophilisation solvent in the absence of excipients, polymers, tensoactives, lipidic or proteic compounds. Besides, said aqueous solution of step c) comprises water, acid and at least one tensoactive, and also a lyophilisation excipient which only grants structure to the lyophilisation cake.

In addition, said procedure comprises a step in which sterilisation is performed by sterilising filtration through a sterilising membrane. Said filtration is performed on the solution obtained in step a), on the solution obtained in step c) or on both.

Said procedure allows the elaboration of said solid composition which contains less than 0.5% of organic solvent, preferably less than 0.1% of organic solvent; preferably said solid composition is essentially free from organic solvent. Additionally, said solid composition is soluble in aqueous solution in the absence of an organic solvent, and has an apparent density from 0.05 g/ml to 0.45 g/ml, preferably from 0.10 g/ml to 0.35 g/ml.

A further object of the present invention is to provide a pharmaceutical formulation of a taxane, suitable to be administered as injectable solutions to mammals, preferably humans, comprising said solid composition and a solubilising composition of said solid composition, which is an aqueous solution in the absence of an organic solvent, preferably an aqueous solution of a tensoactive, preferably Solutol™ HS15.

Another object of the present invention is to provide a kit of elements which comprises a first container holding said lyophilised taxane solid composition; a second container holding said solubilising composition of said taxane solid composition; and a syringe. In a preferred embodiment of this present invention said syringe is prefilled and comprises said first container and said second container.

A further object of the present invention is a kit for the preparation of the injectable taxane formulation suitable to prepare parenteral infusion solutions for mammals, preferably humans, comprising said taxane lyophilised solid composition; a solubilising composition of said solid composition of said taxane; a syringe that allows mixing said solubilising composition with said solid composition of said taxane and obtaining a transparent and stable solution of taxane. Wherein said tensoactive is selected from the group consisting of macrogol hydroxistearate, poloxamer, polyvinylpirrolidone or mixtures thereof, preferably Solutol™ HS15; said acid is selected from the group consisting of citric acid, lactic acid, tartaric acid, ascorbic acid, chlorhydric acid and the mixtures thereof, preferably citric acid; said excipient of liophylisation is selected from the group consisting of mannitol, Lutrol™ F68, sorbitol, lactose, glucose, povidone, xilitol, Kollidon™ 17PF or 12PF (K17PF or K12PF); and said taxane is selected from the group comprised by baccatin III derivatives, derivatives from 10-deacetylbaccatin III and conjugates, salts, hydrates and solvates thereof; preferably docetaxel, its salts, hydrates or solvates thereof.

### Detailed description of the invention

In this present invention "taxane" is understood as the compounds extracted from the leaves and bark of the tree commonly known as European yew *(Taxus Baccata* and other species of the Taxus family) as paclitaxel, as well as semi-synthetic products obtained from baccatin III or from 10-deacetylbaccatin III which are also extracted from yew, like docetaxel. Synthetic and semi-synthetic analogous, conjugates, its derivatives, salts, hydrates and solvates of docetaxel and paclitaxel are also comprised in the definition of taxane. Those taxanes obtained from biotechnological processes are also included in said definition. The medical uses of taxanes are varied; their anti-tumoral effects can be mentioned among others. Some examples of cancer which can be treated with docetaxel are: locally advanced or metastatic breast cancer, non-small cell lung cancer, hormone-refractory prostate cancer and ovarian cancer, and gastric cancer.

In this present invention "lyophilisation organic solvent" is understood as an organic solvent which has a relatively high fusion point (above -10°C) to allow solidification and further lyophilisation, but lower than 25°C to allow its work as a liquid at ambient temperature. Among others, acetic acid, dioxane and dimethylsulphoxide comprise a group which can be used to realise the present invention. Said solvents allow an easy and fast dissolution of drugs which are poorly soluble in water, mainly taxanes, without the need of heating and without precipitation within long periods; particularly said solvents allow dissolving docetaxel and paclitaxel. The mixtures of said organic lyophilisation solvents are also considered organic lyophilisation solvents.

"Essentially free from organic solvent" means free from organic solvents such as ethanol added to improve solubilisation, and free from organic lyophilisation solvents. It should be noticed that the phrase "essentially free from organic solvent" makes reference to the fact that said organic lyophilisation solvents are not detectable by gaseous chromatography with detection by mass spectrometry, which is able to detect the presence of said solvents starting from a concentration of 0.003%.

Terbutanol is mentioned in the state of the art as a solvent which might be useful to solubilise taxanes. It is true that in mixtures with organic lyophilisation solvents this is possible, but laboratory tests have proved that this solvent does not achieve solubilisation taxane concentrations easily and completely, especially docetaxel and paclitaxel, required for the present invention. Acetic acid and dioxane are preferred for the present invention.

The tensoactives suitable to be used in the present invention are comprised by the following list: polyetylglycol, polyvinylpirrolidone, poloxamer, hydroxypropylcellulose, polymethacritates, polysine, poly vynil alcohol, poly acrylic acid, ethylene polyoxide, hyaluronic acid, dextrane sulphate and its derivatives, calcium stearate, glyceron monostearate., cetoestearilic alcohol, emulsifying wax of cetomacrogol, sorbitan esters, alquilic eter of ethylene polyoxide, macrogol esters, as cetomacrogol 1000, derivates of ricine oil polyocyothylenic, polysorbates, polysorbate 80, fat acid esters of polyoxyethylenesorbitane (TWEEN™), estearates of polyoxyothylene, sodic dodecilsulphate, calcium carboxymetylcellulose, sodium carboxymetylcellulose, methylcellulose phtalate of hydroxipropylmetylcellulose, non crystalline cellulose, Triton.

A preferred embodiment of the present invention comprises as tensoactives macrogol hydroxistearate, poloxamer, polyvinylpirrolidone, povidone or mixtures thereof. In particular, Lutrol™ F68, Solutol™ HS 15 (polyethylene glycol-15-hydroxystearate), Kollidon™ 17PF or 12PF (K17PF or K12PF) provided by BASF, or mixtures thereof, are preferred. Solutol™ HS15 is macrogol hydroxistearate. It is also known as Polyethylene glycol-15-hydroxystearate, Polyethylene glycol 660 12-hydroxystearate, Macrogol-15-hydroxystearate, CAS No:70142-34-6. It is used in injectables to solubilise hydrophobic actives and avoid sedimentation and recrystallisation.

Its low toxicity and extraordinary solubilising power, allow its use in high concentrations. A very low histaminic release has been proved after the administration to mammals as compared to polysorbates ("Application of Solutol™ HS15- A Potent Solubiliser with a Low Toxicity" F. Ruchatz). Some studies suggest that this solubiliser can cause as a desired side effect the reversion of multiple resistance of certain carcinogenic cells as regards anticancer drugs. (K. H. Frömming et al., Acta Pharm. Technol. 36(4). 1990, 214-220; J.S. Coon et al., Cancer Res. 51 (3), 1991, 897-902; J.S. Coon, Proc. Am. Assoc. Cancer Res. 33, 1992, 484; D. Hoover et al., Fundam. Appl. Toxicol. 14 (1990), 589 pp.).

The apparent density of the lyophilisate is defined as the quotient between the mass of the lyophilisation cake in grams and its volume in milliliters.

The present invention provides a solid composition of lyophilised taxane suitable to prepare pharmaceutical formulations for mammals, mainly humans, having chemical stability which allows storage at ambient temperature in tropical and subtropical climates, which facilitates the operations for the preparation of the infusion for perfusion, is free from tensoactives and toxic excipients, particularly free from polyoxyethylated castor oil and polysorbate 80. Said composition allows oncological treatment in patients with hypersensitivity to said tensoactives, patients suffering from renal illnesses such as saline retention syndrome, the elderly and patients with cardiopathies, etc.

Also, said solid composition of lyophilised taxane is soluble in aqueous solutions, particularly water; is essentially free from organic solvents and has chemical stability due to the presence of acid in its formulation.

The procedure of the present invention, through which said solid composition of lyophilised taxane can be manufactured, consists of dissolving a taxane in organic solvent of lyophilisation and subjecting this dissolution to a first lyophilisation. The resulting cake, which contains a concentration of organic solvent of lyophilisation of up to 8% w/w, is later reconstituted with an aqueous solution and subjected to a second lyophilisation.

In brief, said procedure to manufacture said solid composition of lyophilised taxane comprises the following steps:
a) dissolving a taxane in organic solvent of lyophilisation
b) lyophilising
c) dissolving the lyophilised taxane from the previous step in an aqueous solution
d) lyophilising the taxane aqueous solution obtained in step c).

The present invention proposes an innovative double-lyophilisation method starting from a solution of taxane in an organic solvent of lyophilisation, in which said solution is obtained without the need of external means, neither mechanical nor thermal, to achieve a fast and complete dissolution. Said solution is subjected to a first bulk lyophilisation in a lyophiliser of production of active pharmaceutical ingredients (API). Thus, a solid lyophilised cake is obtained, containing only said taxane and the remains of the lyophilising organic solvent: this solid is the intermediate lyophilised taxane. Therefore, by means of a very simple procedure, a pharmaceutical active of a large specific area and extraordinarily improved solubility is obtained. Said intermediate lyophilised taxane obtained is solubilised in aqueous solution of tensoactive, in the presence of acid and a lyophilisation excipient, and is subjected to a second lyophilisation in a pharmaceutical lyophiliser of final product in vials. Thus the solid composition of lyophilised taxane of the invention is obtained. The second lyophilisate does not require any special infrastructure to process such aqueous solution, for said solution is free from organic solvents.

Said lyophilising organic solvents generate a physically stable solution, and the chemical stability of said taxanes is not affected. It has been demonstrated that the lyophilisation cake obtained from a solution which only contains a lyophilising organic solvent of the characteristics already mentioned, in the absence of water, tensoactives and any excipient is extraordinarily soluble in aqueous solutions of tensoactives.

The solubilisation of said intermediate lyophilised taxane in aqueous solution of a tensoactive is possible due to the particular characteristics of said intermediate lyophilised taxane: large superficial area, apparent density lower than 0.1, preferably between 0.004 and 0.05 g/ml, and the absence of other components. Taxanes such as paclitaxel or docetaxel commercially available as API are not soluble in aqueous solutions in the absence of added organic solvents. The procedure of the invention allows decreasing residual lyophilisation organic solvents in the final product due to the second lyophilisation in which the solvent is water. The contents of residual solvents achieved in the second lyophilisation are lower than 0.5% as for docetaxel, preferably lower than 0.1%. More preferably, a solid composition of lyophilised taxane essentially free from organic solvent is obtained.

The intermediate lyophilised taxane is dissolved in aqueous solutions of polymers or a mixture of said polymers, namely Lutrol^{™} F68, Solutol^{™} HS15, povidone K12PF or K17PF. The addition of ethanol may make the dissolution easier, though it generally decreases physical stability; therefore it is preferred not to use it.

Said aqueous solutions of taxane prepared with Solutol^{™} HS 15 at different concentrations, have the drawback of not being able to be lyophilised directly, due to the fact that this polymer is a solid of a low range of liquefaction (30-40°C) which, at the final stages of lyophilisation, loses its structure and the lyophilisate ends in a compact structure which cannot be completely dried nor readily redissolved in order to obtain the premix solution. The inconvenient is solved by adding a solid lyophilisation excipient of a higher fusion point like mannitol, Lutrol^{™} F68, sorbitol, lactose, glucose, povidone, xilitol, Kollidon^{™} 17PF and 12PF (K17PF and K12PF) or other, so that the resulting solution, when lyophilised, maintains its spongy structure thus allowing an easy dissolution of the solid composition of lyophilised taxane of the invention, even using only water to solubilise the solid composition of lyophilised taxane for the preparation of the premix solution which is injected to the perfusion solution.

The apparent density of the lyophilisates has been carefully assessed, and after a great number of experiments the apparent density values in which the technical effects of the invention are possible -such as easy reconstitution of said solid composition of lyophilised taxane in an aqueous solution free from organic solvent- were obtained. It has been proved that the solubility of the solid composition of the invention improves as its apparent density decreases. In other words, the lower the apparent density of the lyophilisation cake, the fastest its dissolution. Also, if the apparent density of the solid composition of lyophilised taxane of the present invention is very low, the concentration of the tensoactive is not enough to maintain said taxane in solution, and said taxane precipitates rapidly.

Said values of apparent density are, for the intermediate lyophilised taxane, lower than 0.1g/ml, preferably from 0.004 g/ml to 0.05 g/ml, most preferably from 0.006 g/ml to 0.02 g/ml; while for the solid composition of lyophilised taxane of the present invention the values of apparent density range from 0.10g/ml to 0.45 g/ml, preferably between 0.15 g/ml and 0.35 g/ml.

When said intermediate lyophilised taxane, preferably prepared with docetaxel, is dissolved in said aqueous solution of tensoactive, the pH of the solution obtained, which contains Solutol^{™}, is between 6.06 and 7.70. It is observed a high level of chemical degradation of the taxanes when the pH is maintained at values higher than 6, in particular of docetaxel, which degrades mainly to 7-epidocetaxel, in a percentage which may reach 60% under the conditions described.

To solve this technical problem, acid is added to the aqueous solution of the tensoactive and the intermediate lyophilised taxane is then dissolved in the same solution. The addition of acid supplies chemical stability to the solid composition of lyophilised taxane of the invention.

The addition of acid has proved to remarkably increase the stability of taxane to obtain said solid composition of lyophilised taxane with a purity higher than 99% in the case of docetaxel. Among the great quantity of acids which can be used to implement the present invention, it can be mentioned: malic acid, tartaric acid, hydrochloryc acid, citric acid, etc. Any person skilled in the art may easily determine the use of any other acid which is useful for pharmaceutical products. Preferably, citric acid in a quantity greater than 20mg per gram of Solutol™ HS 15 is used.

The procedure of the present invention also comprises a step in which sterilisation is performed by filtration through a sterilising membrane. Said filtration is performed on the solution of lyophilising organic solvent obtained in step a), on the aqueous solution obtained in step c) or on both.

Said sterilising filtration is performed using 0.2 or 0.22 micron filtres. Said filtration is done prior to lyophilisation, thus obtaining a sterile powder as the solid composition of the invention. The solubilising composition is also sterilised by filtration. Therefore, the sterilisation of solids which involves complex and costly processes and poses risks to the drugs is avoided.

The present invention also provides a pharmaceutical formulation comprising said solid composition of lyophilised taxane and a solubilising composition which are mixed prior to their use, resulting in a transparent solution.

In a preferred embodiment, the present invention comprises a kit consisting of a first container holding a solid composition of lyophilised taxane of the invention; a second container holding the solubilising composition of the invention, and a syringe.

In another preferred embodiment, said syringe is prefilled and comprises said first and second containers, independent from one another, with means for connecting said containers prior to the administration and, optionally, with a filter.

In a preferred version of the invention said syringe is prefilled and comprises said sterile compartments. Therefore, the preparation of the perfusion formulation is simplified since both compartments are brought into contact, and by means of a gentle motion the dissolution of the solid composition of lyophilised taxane is reached in said solubilising composition. Finally, this is injected into the perfusion bag or flask containing saline or dextrose solution. Also, said prefilled syringe allows decreasing the preparation time of the perfusion formulation remarkably, eliminating a significant percentage of the contamination risks for the product as well as for the health care providers and patients, as it happens with the product currently available.

The pharmaceutical perfusion solution formulated with the solid composition of lyophilised taxane of the present invention contains less than 1 mg/ml of taxane in normal saline or dextrose solution. It also contains a tensoactive, preferably Solutol™ essentially free from organic solvent.

Also, said pharmaceutical perfusion solution for the infusion of taxanes in mammals, mainly humans, for the treatment of cancer, requires shorter times for the perfusion process (lower than 30 minutes) and it does not require pretreatment with steroids or antihistamines, since it is free from polysorbate 80, polyoxyethylated castor oil, emulsions and liposomes.

The shortening of operative times for the preparation of the perfusion solution, as well as the shortening of the perfusion times during the administration of the formulation of the invention grants the following benefits: more patients assisted in the systems of day hospital of the departments of clinical oncology of the current system of health (public and private sectors); reduced operative costs on the basis of pharmacoeconomy, due to a better use of sanitary resources; prioritisation of the safety of health care workers involved in said manipulation (the risk of contact of the initial dilution solution with the skin and the mucosa of the operation is lowered to almost 0).

It is worth highlighting that other drugs which are susceptible of being used as active pharmaceutical ingredient instead of the taxanes described to realise the present invention are: albuterol, adapalene, doxazosin mesylate, mometasone furoate, ursodiol, amphotericin, enalapril maleate, felodipine, nefazodone hydrochloride, valrubicin, albendazole, conjugated estrogens, medroxyprogesterone acetate, nicardipine hydrochloride, zolpidem tartrate, amlodipine besylate, ethinyl estradiol, omeprazole, rubitecan, amlodipine besylate/benazepril hydrochloride, etodolac, paroxetine hydrochloride, atovaquone, felodipine, podofilox, paricalcitol, betamethasone dipropionate, fentanyl, pramipexole dihydrochloride, Vitamin D and related analogues, finasteride, quetiapine fumarate, alprostadil, candesartan, cilexetil, fluconazole, ritonavir, busulfan, carbamazepine, flumazenil, risperidone, carbidopa/levodopa, ganciclovir, saquinavir, amprenavir, carboplatin, glyburide, sertraline hydrochloride, rofecoxib carvedilol, halobetasolproprionate, sildenafil citrate, celecoxib, chlorthalidone, imiquimod, simvastatin, citalopram, ciprofloxacin, irinotecan hydrochloride, sparfloxacin, efavirenz, cisapride monohydrate, lansoprazole, tamsulosin hydrochloride, mofafinil, azithromycin, clarithromycin, letrozole, terbinafine hydrochloride, rosiglitazone maleate, diclofenac sodium, lomefloxacin hydrochloride, tirofiban hydrochloride, telmisartan, diazapam, loratadine, toremifene citrate, thalidomide, dinoprostone, mefloquine hydrochloride, trandolapril, docetaxel, mitoxantrone hydrochloride, tretinoin, etodolac, triamcinolone acetate, estradiol, ursodiol, nelfinavir mesylate, indinavir, beclomethasone dipropionate, oxaprozin, flutamide, famotidine, nifedipine, prednisone, cefuroxime, lorazepam, digoxin, lovastatin, griseofulvin, naproxen, ibuprofen, isotretinoin, tamoxifen citrate, nimodipine, amiodarone, and alprazolam, amphotericin B, cyclosporine, etoposide, topotecan, melphalane, idarubicine, doxorubicin, vinorelbine, vinblastine, vinchristine. Reference is made herein to weight/weigh (w/w) concentrations, when no explicit reference is made to other kind of magnitudes.
For a better insight into the technical and functional aspects of the present invention, and without restricting the scope of this present patent application, there follows a set of examples of application involving some of the alternatives comprised herein, and a set of comparative examples which allow assessing the emergent differences regarding the prior state of the art.

### EXAMPLES

Materials: In the experiments glass vials type I, 20mm-wide mouth, 22mm body diameter, 27 mm of height, Nuova Ompi, with a nominal capacity of 7 ml were used. The 20 mm apyrogen caps for lyophilisation were sterilized with butyl-bromide from Helvoet Pharma. The solvents used were PA quality, acetic acid, Merk, item 1.000632511; TEDIA Dioxane, item DR-0480; Merck ethanol, item 1.009832511. The distilled water used was water quality for injectables (WFI) according to USP and EP specifications. BASF Solutol™ HS15, item No: 51633963; BASF Lutrol™ F68 (Poloxamer 188), item No: 51633115; BASF Lutrol™ E400, item No:51632267, BASF Kollidon™ item No:51598188.The lyophilisation assays were carried out in a VIRTIS ADVANTAGE lyophiliser driven by a MENTOR unit. The determinations of HPLC were performed either in a HPLC BECKMAN System Gold with solvent module model 118, diodes array detector Model 116 and auto-injector Wilson model 234, or in a HPLC Waters model 1525 with binary pumps and a diode array detector, model 2996, a forced circulation oven Alltech (column thermostat Jetstream 2 Plus) and automatic sampler Waters 717 Plus. The solvent content was analysed by gaseous chromatography AGILENT TECHNOLOGIES 6890N GC system with injector AGILENT TECHNOLOGIES 7683 B series with a sampler PERKIN ELMER Head Space Turbo Matrix 40 and a Mass Selective Detector AGILENT TECHNOLOGIES 5973 Network Mass selective Detector.

### Example 1

Docetaxel (API commercially available) in a quantity of 6.624g was placed into a 500ml container, and dissolved with 275 ml of dioxane. Once dissolved, it was lyophilised following a cycle of freezing stages of -60°C for 240 min, and lyophilisation at - 3°C during 1500 min, at 10°C during 1500 min and final drying at 30°C during 1700 min at 30°C. The intermediate lyophilised taxane obtained in a quantity of 6.903 g resulted in a homogeneous solid of an apparent density of 0.025 g/ml and a content of residual dioxane of 8.6%, measured by gaseous chromatography and detected by mass spectrometry. The purity of docetaxel of said intermediate lyophilised taxane measured by HPLC was determined at 99.69% measured as area percentage, detected by UV at 232 nanometers, using a stainless steel column Waters Symmetry C18, of 4.6 mm x 15 cm, 5 microns and a mobile phase of acetonitrile:methanol:water (26:32:42, v:v:v) filtered and degassed.

### Example 2

Anhydrous docetaxel (API commercially available), purity 98.2%, anhydrous base, in a quantity of 2.319g was placed into a 250ml container, and dissolved with 160 ml of acetic acid. Once dissolved, it was sterilised by means of a 0.22 micron filter and filtered following a cycle of lyophilisation with freezing stage of -60°C during 240 min, and lyophilisation at -5°C during 1500 min, at 10°C during 1500 min and final drying at 25°C during 380 min and 1470 min at 30°C. The sterilised intermediate lyophilised taxane, obtained in a quantity of 2.18g resulted in a homogeneous solid, with an apparent density of 0.014 g/ml and a residual acetic acid content of 0.8% measured by gaseous chromatography and detected by mass spectrometry. The purity of lyophilised docetaxel measured by HPLC was determined at 99.35%, measured as area percentage, detected by UV at 232 nanometers, using a stainless steel column Waters Symmetry C18, of 4.6 mm x 15 cm, 5 microns and a mobile phase of acetonitrile:methanol:water (26:32:42, v:v:v) filtered and degassed.

### Example 3

Anhydrous docetaxel (API commercially available), anhydrous base, purity 98.3%, in a quantity of 0.874g was placed into a 100ml container, and dissolved with 64 ml of acetic acid. Once dissolved, it was lyophilised following a lyophilisation cycle with a freezing stage of -60°C for 240 min, and lyophilisation at -5°C during 2400 min, at 10°C during 1500 min and final drying at 30°C during 1490 min. The intermediate lyophilised taxane obtained in 41 samples in vials of 20 mg of docetaxel each was a homogeneous solid. The purity of said lyophilised docetaxel measured by HPLC was 99.24% as area percentage, detected by UV at 232 nanometers, using a stainless steel column Waters Symmetry C18, of 4.6 mm x 15 cm, 5 microns and a mobile phase of acetonitrile:methanol:water (26:32:42, v:v:v) filtered and degassed. Subsequently, 8 of said vials dissolved easily in a volume of 2 ml of an aqueous solution composed of Solutol™ HS15:povidone (K17PF):water (25:5:70). Once dissolved said vials are pre-capped and taken to lyophilisation following a lyophilisation cycle with freezing stage of -60°C for 240 min, and lyophilisation at -3°C during 1500 min, at 5°C during 1500 min and final drying at 25°C during 2910 min. A solid composition of lyophilised taxane is obtained in the absence of acid with a purity of docetaxel of 73.76% measured as area percentage, detected by UV at 232 nanometers, in HPLC using a stainless steel column Waters Symmetry C18, of 4.6 mm x 15 cm, 5 microns and a mobile phase of acetonitrile:methanol:water (26:32:42, v:v:v) filtered and degassed.

### Example 4

The intermediate lyophilised taxane (docetaxel) obtained in Example 1 was placed in a quantity of 446 mg in a 100ml-container, and it was readily dissolved in a final volume of 40ml of a mixture comprising Solutol™ HS 15:mannitol:citric acid, water (25:5.0.525:69.475). Once dissolved, it was sterilised by means of sterilising filtration with a 0.22 micron filtre. A quantity of 2ml of this solution was dosed in 7ml vials, which resulted in 18 doses of 20mg of docetaxel each. Said vials were pre-capped and taken to lyophilisation following a lyophilisation cycle with freezing stage of -60°C during 240 min, and lyophilisation at -5°C during 1500 min, at 10°C during 1500 min and final drying at 25°C during 2120 min. A solid composition of lyophilised taxane of the invention, sterile, as a homogeneous cake of an apparent density of 0.32 g/ml is obtained. The purity of the docetaxel obtained through this lyophilisation process is of 99.54% measured as area percentage, detected by UV at 232 nanometers, in HPLC using a stainless steel column Waters Symmetry C 18, of 4.6 mm x 15 cm, 5 microns and a mobile phase of acetonitrile: methanol:water (26:32:42, v:v:v) filtered and degassed. The residual dioxane content was non detectable, as measured by gaseous chromatography and detection by mass spectroscopy.

### Example 5

The intermediate lyophilized taxane (docetaxel)obtained in Example 1 is placed in a quantity of **446** mg in a 100ml-container, and it is readily dissolved in a final volume of 40 ml of a mixture comprising Solutol^{™} HS15:mannitol:citric acid, water (25:5.0.65:69.35). Once dissolved, it is sterilized by filtration (0.2 micron membrane). A quantity of 2 ml of this solution is dosed in 7 ml vials, resulting in 18 doses of 20 mg of docetaxel each, which are pre-capped and taken to lyophilisation following a lyophilisation cycle with freezing stage of -60°C for 240 min, and lyophilisation at -5°C during 1500 min, at 10°C during 1500 min and final drying at 25°C during 2120 min. A solid composition of lyophilised taxane of the invention, sterile, as a homogeneous cake of an apparent density of 0.32 g/ml is obtained. The purity of docetaxel obtained through this lyophilisation process is of 99.70% measured as area percentage, detected by UV at 232 nanometers, in HPLC using a stainless steel column Waters Symmetry C18, of 4.6 mm x 15 cm, 5 microns and a mobile phase of acetonitrile:methanol:water (26:32:42, v:v:v) filtered and degassed. The residual dioxane content was non detectable, as measured by gaseous chromatography and detection by mass spectroscopy. The lyophisate is dissolved in 4 ml of water for injectables, resulting in a solution of pH=2.8.

### Example 6

The intermediate lyophilised taxane (docetaxel) obtained in Example 1 was placed in a quantity of **1117** mg in a 250ml-container, and it was readily dissolved in a final volume of 150 ml of a mixture comprising Solutol™HS15:mannitol:citric acid, water (16.7:3.3:0.43:79.57). Once dissolved, it was sterilised through a 0.22 micron filtre. A quantity of 3 ml of this solution was dosed in 7ml vials, which resulted in 48 doses of 20mg of docetaxel each. Said vials were pre-capped and taken to lyophilisation following a lyophilisation cycle with freezing stage of -60°C during 240 min, and lyophilisation at -5°C during 1500 min, at 10°C during 1500 min and final drying at 25°C during 5100min. A sterile solid composition of lyophilised taxane of the invention is a homogeneous cake of an apparent density of 0.21 g/ml is obtained. The purity of the docetaxel obtained through this lyophilisation process is of 99.64% measured as a area percentage, detected by UV at 232 nanometers, in HPLC using a stainless steel column Waters Symmetry C18, of 4.6 mm x 15 cm, 5 microns and a mobile phase of acetonitrile:methanol:water (26:32:42, v:v:v) filtered and degassed. Humidity content was 0.07, the residual dioxane content was non detectable, as measured by gaseous chromatography and detection by mass spectroscopy.

### Example 7

The intermediate lyophilized taxane (docetaxel) obtained in Example 2 is placed in a quantity of **447** mg in a 100ml-container, and it is readily dissolved in a final volume of 40 ml of a mixture comprising Solutol™ HS 15:mannitol:citric acid, water (25:5:0.525:69.475). Once dissolved, it is sterilized by filtration. A quantity of 2 ml of this solution is dosed in 7 ml vials, resulting in 18 doses of 20 mg of docetaxel each, which are pre-capped and taken to lyophilisation following a lyophilisation cycle with freezing stage of -60°C for 240 min, and lyophilisation at -5°C during 1500 min, at 10°C during 1500 min and final drying at 25°C during 2120 min. A solid composition of lyophilised taxane of the invention, sterile, as a homogeneous cake of an apparent density of 0.32 g/ml is obtained. The purity of docetaxel obtained through this lyophilisation process is of 99.46% measured as area percentage, detected by UV at 232 nanometers, in HPLC using a stainless steel column Waters Symmetry C18, of 4.6 mm x 15 cm, 5 microns and a mobile phase of acetonitrile:methanol:water (26:32:42, v:v:v) filtered and degassed.

### Example 8

The intermediate lyophilized taxane (docetaxel) obtained in Example 2 is placed in a quantity of **446** mg in a 100 ml-container, and it is readily dissolved in a final volume of 40 ml of a mixture comprising Solutol® HS 15:mannitol:citric acid, water (25:5:0.65:69.35). Once dissolved, it is sterilized by filtration (0.2 micron membrane). A quantity of 2 ml of this solution is dosed in 7 ml vials, resulting in 18 doses of 20 mg of docetaxel each, which are pre-capped and taken to lyophilisation following a lyophilisation cycle with freezing stage of -60°C for 240 min, and lyophilisation at -5°C during 1500 min, at 10°C during 1500 min and final drying at 25°C during 2120 min. A solid composition of the invention is sterile, a homogeneous cake of an apparent density of 0.32 g/ml. The purity of docetaxel obtained through this lyophilisation process is of 99.65% measured as area percentage, detected by UV at 232 nanometers, in HPLC using a stainless steel column Waters Symmetry C18, of 4.6 mm x 15 cm, 5 microns and a mobile phase of acetonitrile: methanol: water (26:32:42, v:v:v) filtered and degassed.

## Claims

1. A lyophilised solid composition of taxane, suitable for the preparation of pharmaceutical formulations for mammals, in particular humans, comprising said taxane, tensoactive, lyophilisation excipient and acid, and it is essentially free from organic solvent.

2. The composition of claim 1, wherein said lyophilised solid composition of taxane has an apparent density between 0.05 g/ml and 0.45 g/ml.

3. The composition of claim 1, wherein said lyophilised solid composition of taxane has an apparent density between 0.10 g/ml and 0.35 g/ml.

4. The composition of claim 1, wherein said lyophilised solid composition of taxane is sterile and soluble in aqueous solutions in the absence of an organic solvent.

5. The composition of claim 1, wherein said tensoactive is selected from the group consisting of macrogol hydroxistearate, poloxamer, polyvinylpirrolidone or mixtures thereof.

6. The composition of claim 1, wherein said lyophilised solid composition of taxane is free from polysorbate 80 and polyoxyethylated castor oil.

7. The composition of claim 1, wherein said tensoactive is Solutol^{™} HS15.

8. The composition of claim 1 wherein said acid is selected from the group consisting of citric acid, lactic acid, tartaric acid, ascorbic acid, hydrochloric acid and the mixtures thereof.

9. The composition of claim 1 wherein said excipient of lyophylisation is selected from the group consisting of mannitol, Lutrol™ F68, sorbitol, lactose, glucose, povidone, xilitol, Kollidon™ 17PF or 12PF (K17PF or K12PF).

10. The composition of claim 1, wherein said taxane is selected from the group comprised by baccatin III derivatives, derivatives from 10-deacetilbaccatin III and conjugates, salts, hydrates and solvates thereof.

11. The composition of claim 1, wherein said taxane is docetaxel, its salts, hydrates or solvates thereof.

12. The composition of claim 1, wherein said taxane is paclitaxel, its salts, hydrates or solvates thereof.

13. A procedure for the preparation of the composition of claim 1, comprising the following steps:
a) dissolving a taxane in lyophilisation organic solvent;
b) lyophilizing;
c) dissolving the intermediate lyophilised taxane from the previous step in an aqueous solution and
d) lyophilising the aqueous solution of taxane obtained in step c).

14. The procedure of claim 13, wherein said lyophilisation organic solvent is selected from the group consisting of dioxane, acetic acid, dymetylsuphoxide, and the mixtures thereof.

15. The procedure of claim 13, wherein the taxane from step a) dissolves in said organic solvent of lyophilisation in the absence of excipients, polymers, tensoactives, lipidic or proteic compounds.

16. The procedure of claim 13, wherein the aqueous solution from step c) comprises water, acid and at least one tensoactive.

17. The procedure of claim 13, wherein the aqueous solution from step c) also comprises a lyophilisation excipient.

18. The procedure of claim 17, wherein said lyophilisation excipient only provides the lyophilisation cake with a structure.

19. The procedure of claim 17, wherein said lyophilisation excipient is selected from the group consisting of mannitol, Lutrol™ F68, sorbitol, lactose, glucose, povidone, xilitol, Kollidon™ 17PF or 12PF (K17PF or K12PF).

20. The procedure of claim 16, wherein said acid is selected from the group consisting of citric acid, lactic acid, tartaric acid, ascorbic acid, hydrochloric acid and the mixtures thereof.

21. The procedure of claim 13, which is also sterilised by filtration through a sterilising membrane.

22. The procedure of claim 21, wherein said filtration is applied to the solution obtained in step a).

23. The procedure of claim 21, wherein said filtration is applied to the solution obtained in step c).

24. The procedure of claim 13, wherein said solid composition contains less than 0.5% of organic solvent.

25. The procedure of claim 13, wherein said solid composition contains less than 0.1% of organic solvent.

26. The procedure of claim 13, wherein said solid composition is essentially free from lyophilisation organic solvent.

27. The procedure of claim 13, wherein said solid composition is soluble in aqueous solution in the absence of an organic solvent, and has apparent density from 0.05 g/ml to 0.45 g/ml.

28. The procedure of claim 13, wherein said solid composition is soluble in aqueous solution in the absence of an organic solvent, and has apparent density from 0.10 g/ml to 0.35 g/ml.

29. The procedure of claim 13, wherein said taxane is selected from the group consisting of baccatin III derivatives, 10-deacetylbaccatin III derivatives, conjugates, salts, hydrates and solvates thereof.

30. The procedure of claim 13, wherein said taxane is docetaxel, its salts, hydrates or solvates thereof.

31. The procedure of claim 13, wherein said taxane is paclitaxel, its salts, hydrates or solvates.

32. A pharmaceutical formulation of a taxane suitable to be injected as a solution to mammals, preferably humans, comprising a solid composition according to any of the claims from 1 to 12, and a solubilising composition of said solid composition.

33. The formulation of claim 32, wherein said taxane is selected from the group consisting of baccatin III derivatives, 10-deacetylbaccatin III derivatives, conjugates, salts, hydrates and solvates thereof.

34. The formulation of claim 32, wherein said taxane is docetaxel, its salts, hydrates or solvates thereof.

35. The formulation of claim 33, wherein said taxane is paclitaxel, its salts, hydrates or solvates thereof.

36. The formulation of claim 32, wherein said solubilising composition is an aqueous solution in the absence of an organic solvent.

37. The formulation of claim 32, wherein said solubilising composition is an aqueous solution of a tensoactive.

38. The formulation of claim 32, wherein said solubilising composition is an aqueous solution of Solutol® HS 15.

39. A kit comprising a first container holding a solid composition of lyophilised taxane as claimed in any of the claims from 1 to 12; a second container holding a solubilising composition of said solid composition of taxane; and a syringe.

40. The kit according to claim 39 wherein said syringe is prefilled and comprises said first container and said second container.

41. A kit for the preparation of an injectable formulation of taxane, suitable to prepare parenteral infusion solutions for mammals, preferably humans, comprising a solid composition of said lyophilised taxane as any of the claims between 1 and 12; a solubilising composition of said solid composition of said taxane; a syringe to mix said solubilising composition with said solid composition of said taxane to obtain, therefore, a transparent and stable solution of taxane.
